(19) Europäisches Patentamt
European Patent Office
Office européen des brevets

(11) **EP 2 154 529 B1**

(12) **EUROPÄISCHE PATENTSCHRIFT**

(45) Veröffentlichungstag und Bekanntmachung des Hinweises auf die Patenterteilung:
**18.12.2013 Patentblatt 2013/51**

(51) Int Cl.:
*G01N 33/00* (2006.01)  *G08B 17/10* (2006.01)

(21) Anmeldenummer: **09008057.3**

(22) Anmeldetag: **19.06.2009**

(54) **Verfahren zur Auswertung von Gassensorsignalen**

Method for evaluating gas sensor signals

Procédé d'évaluation de signaux de capteurs de gaz

(84) Benannte Vertragsstaaten:
**AT BE BG CH CY CZ DE DK EE ES FI FR GB GR HR HU IE IS IT LI LT LU LV MC MK MT NL NO PL PT RO SE SI SK TR**

(30) Priorität: **05.08.2008 DE 102008036436**

(43) Veröffentlichungstag der Anmeldung:
**17.02.2010 Patentblatt 2010/07**

(73) Patentinhaber: **Hekatron Vertriebs GmbH 79295 Sulzburg (DE)**

(72) Erfinder:
• **Lacoste, Thilo**
  **79282 Ballrechten-Dottingen (DE)**
• **Weidner, Martin**
  **79379 Mülheim (DE)**

(74) Vertreter: **Maucher, Wolfgang et al Patent- und Rechtsanwaltssozietät Maucher, Börjes & Kollegen Urachstrasse 23 79102 Freiburg im Breisgau (DE)**

(56) Entgegenhaltungen:
• **M SPEARPOINT: "Experimental analysis of the performance of a multisensor two-stage fire detection and water discharge algorithm", FIRE SAFETY JOURNAL, Bd. 29, Nr. 2-3, 1. Oktober 1997 (1997-10-01), Seiten 141-157, XP55002522, ISSN: 0379-7112, DOI: 10.1016/S0379-7112(96)00017-3**
• **EVERETTE S. GARDNER ET AL: "FORECASTING WITH EXPONENTIAL SMOOTHING: SOME GUIDELINES FOR MODEL SELECTION", DECISION SCIENCES, Bd. 11, Nr. 2, 1. April 1980 (1980-04-01), Seiten 370-383, XP55002688, ISSN: 0011-7315, DOI: 10.1111/j.1540-5915.1980.tb01145.x**
• **EVERETTE S. GARDNER: "Exponential smoothing: The state of the art", JOURNAL OF FORECASTING, Bd. 4, Nr. 1, 1. Januar 1985 (1985-01-01), Seiten 1-28, XP55002663, ISSN: 0277-6693, DOI: 10.1002/for.3980040103**
• **E GARDNERJR: "Exponential smoothing: The state of the art-Part II", INTERNATIONAL JOURNAL OF FORECASTING, Bd. 22, Nr. 4, 1. Oktober 2006 (2006-10-01), Seiten 637-666, XP55002644, ISSN: 0169-2070, DOI: 10.1016/j.ijforecast.2006.03.005**
• **'Signal Processing and Data filtering', [Online] 25 Mai 2005, XP055045912 Gefunden im Internet: <URL:http://www.das.ufsc.br/~aarc/ensino/gr aduacao/DAS5901/> [gefunden am 2012-11-28]**

EP 2 154 529 B1

**Beschreibung**

Die Erfindung betrifft ein Verfahren zur Auswertung von Signalen einer Messeinrichtung, die wenigstens einen Gassensor und eine Signalverarbeitungseinrichtung aufweist, welche die Messwerte des wenigstens einen Gassensors verarbeitet. Außerdem betrifft die Erfindung eine Vorrichtung mit welcher das Verfahren ausgeführt werden kann sowie die Verwendung einer solchen Vorrichtung in einem Gefahrenmelder.

Der Einsatz von Sensoren in Gefahrenmeldern, beispielsweise in Brandmeldern zur Identifikation einer potentiellen Gefahr beispielsweise bei Änderung der Konzentration eines oder mehrerer Zielgase ist bekannt. Insbesondere Gassensoren mit einer optimierten Leistungsaufnahme können dabei selbst ohne Einwirkung eines Zielgases eine in Größe und Richtung unbestimmte, fortlaufende Drift zeigen, was eine Absolutmessung der Gaskonzentration, ohne eine geeignete Kompensation der Ursache für die Drift, aufgrund fehlender Null-Referenz unmöglich macht. Aufgrund von Messungen an Testbränden ist es jedoch auch bekannt, dass bei verschiedenen auf Brände hinweisenden Störgrößen die Signaländerungen (Ableitung des Signals) signifikant höher sind als die durch Umweltänderungen (Luftfeuchtigkeit, Temperatur etc.) verursachte Signaldrift. Daher kann die Geschwindigkeit einer Signaländerung (Signalableitung) als Alarmierungs-kriterium herangezogen werden.

In diesem Zusammenhang kennt man aus dem Periodikum "Fire Safety Journal" (Bd.29) durch den Artikel "Experimental Analysis of the Performance of a Multisensor Two-stage Fire Detection and Water Discharge Algorithm" bereits einen Rauchmelder der seine Messsignale mittels einfacher exponentieller Glättung glättet. Die Glättung wird mit zwei verschiedenen Gewichtungswerten durchgeführt, wobei ein Gewichtungswert einer Langzeitglättung entspricht und der andere einer Kurzzeitglättung. Die Differenz aus dem jeweils einfach exponentiell geglätteten Langzeit- und Kurzzeitwert wird mit einer Alarmschwelle verglichen um zu bestimmen, ob eine Alarmsituation vorliegt.

Weiter beschreibt die Monographie "Forecasting with exponential smoothing: Some Guidelines for Model Selection" von E. Gardner verschiedene Modelle zur exponentiellen Glättung. In dieser Abhandlung wird auf einen Nachteil einfach exponentiell geglätteter Signale verwiesen, nämlich dass diese dem ungeglätteten Signal gegenüber nachliefen. Gemäß der Abhandlung wird dieser Nachteil durch eine exponentielle Glättung 2. Ordnung nach Brown abgeschwächt.

Für eine zeitnahe Berechnung der Ableitung eines Signals müsste in der Signalverarbeitungseinrichtung die Differenz aus dem aktuellen und einem zeitlich zurückliegenden Signalwert ermittelt werden. Um hierbei gerade auch schnelle Änderungen erfassen zu können, sollte der zeitliche Abstand dieser beiden Messwerte gering sein, beispielsweise in der Größenordnung von Sekunden. So kann es aufgrund eines starken Rauschens mancher Sensoren und der damit verbundenen starken Schwankungen des Sensorsignals jedoch zu extrem schwankenden Differenzwerten und damit schwankenden Steigungs- bzw. Ableitungswerten führen, die als Alarmierungskriterium ungeeignet wären. Ebenso scheint es zunächst nicht möglich die absoluten Änderungen der interessierenden Messgröße zu erfassen.

Es ist daher die Aufgabe der vorliegenden Erfindung, ein Verfahren zur Auswertung von Sensorsignalen zur Verfügung zu stellen, das kostengünstig, ohne zusätzliche Sensoren zur Signalkompensation, mit geringem Speicher- und Rechenaufwand für die Signalverarbeitungseinrichtung eine Verarbeitung stark schwankender Messwerte erlaubt und mittels der verarbeiteten Messwerte eine Bewertung kurz- und langfristiger Trends sowie der absoluten Änderung der Sensorsignale sogar bei unbekannten funktionellen Zusammenhängen zwischen Signalschwankungen und deren Ursachen gestattet.

Diese zunächst widersprüchlich erscheinende Aufgabe wird gelöst durch ein Verfahren der eingangs genannten Art, welches zumindest die folgenden Verfahrensschritte aufweist:

a) Vorgabe zumindest eines, bevorzugt mehrerer Glättungsparameter $\alpha^i$ ;
b) Erfassung von Messwerten in vorbestimmbaren Zeitintervallen durch die Messeinrichtung, Weiterleitung der Messwerte an die Signalverarbeitungseinrichtung und Verarbeitung der Messwerte in dieser;
c) Berechnung eines Startwertes $y_0$ zu einem nach Beginn der Erfassung liegenden Zeitpunkt $t_0$ beispielsweise als Mittelwert aus mehreren zuvor erfassten Messwerten $y_{t<t0}$ ;
d) Nach dem Ablauf des nächsten Zeitintervalls Erfassung des Messwerts $y_t$ zum Zeitpunkt $t$ ;
e) Anwendung eines Glättungsverfahrens, insbesondere einer exponentiellen Glättung, auf den aktuellen Messwert

$y_t$ zur Ermittlung des geglätteten Wertes erster Ordnung $y_t^{*}$ ;

f) Erneute Anwendung eines Glättungsverfahrens, insbesondere einer exponentiellen Glättung, auf den Wert $y_t^{*}$

zur Ermittlung des geglätteten Wertes zweiter Ordnung $y_t^{**}$ ;

g) Verrechnung der Werte der ersten und zweiten Glättung $y_t^{i*}$ und $y_t^{i**}$ , insbesondere von kurzfristig geglätteten Werten $y_t^{k*}$ und $y_t^{k**}$ , sowie von Glättungsparametern $\alpha^i$, insbesondere des kurzfristigen Glättungsparameters $\alpha^k$, mittels einer Linearkombination zur Steigung (5), insbesondere der kurzfristigen Steigung $Y_t^k$ , im geglätteten Messwert $\hat{y}_t$, (2);

h) Wiederholung der Verfahrensschritte d)-g) für die Messwerte der Zeitpunkte $t+1, t+2, ..., t+n$.

Um aus den stark schwankenden Messwerten $y_t$ weitere Informationen gewinnen zu können, werden diese also mehrfach einer Glättung, bspw. durch so genannte gleitende Mittelwertbildung, vorzugsweise jedoch einer exponentiellen Glättung, unterzogen.

Rechnerisch werden für die exponentielle Glättung die aus den Signalen des oder der Sensoren bestehenden Messwerte den folgenden Algorithmen unterzogen. Aus den Messwerten $y_t$ der ersten Zeitintervalle wird zunächst ein Startwert $y_0$ für die exponentielle Glättung aus den ersten m Messwerten gemäß

$$y_0 = \frac{\sum_{t=1}^{m} y_t}{m} = y_0^* = y_0^{**}$$

gewonnen. Jeder weitere, nach einem kurzen Zeitintervall aufgenommene Messwert $y_t$ wird dann zweifach geglättet, wobei * den Grad der Ordnung anzeigt, nach:

$$y_t^* = \alpha \cdot y_t + (1-\alpha) \cdot y_{t-1}^* = \alpha \cdot (y_t - y_{t-1}^*) + y_{t-1}^*$$

mit $\alpha \ll 1$ (exponentielle Glättung erster Ordnung)
und

$$y_t^{**} = \alpha \cdot y_t^* + (1-\alpha) \cdot y_{t-1}^{**} = \alpha \cdot (y_t^* - y_{t-1}^{**}) + y_{t-1}^{**}$$

(exponentielle Glättung zweiter Ordnung)

Anhand dieser Methode wird mittels eines Glättungsfaktors $\alpha$ der Einfluss älterer Datenwerte auf den aktuellen Wert festgelegt. Je kleiner der Wert des Glättungsparameters, umso stärker ist der Einfluss der vergangenen Daten und umso stärker ist die Glättung. Der geglättete Wert folgt bei Trendänderungen dem ursprünglichen Wert mit einem zeitlichen Abstand. Um dennoch zeitnah Trends erkennen zu können, erfolgt eine zweite exponentielle Glättung (exponentielle Glättung zweiter Ordnung) der einfach geglätteten Werte sowie eine Verrechnung beider Werte aus erster und zweiter Ordnung miteinander. Der neue Datenwert $\hat{y}_t$ enthält deutlich weniger Schwankungen als die Rohdaten, folgt aber Trendänderungen schnell.

Bei zweckmäßigen Varianten des Verfahrens verbleiben nur die geglätteten Werte $y_t^*$ und $y_t^{**}$ in einem der Signalverarbeitungseinrichtung zugeordneten Arbeitsspeicher für die Berechnung der geglätteten Werte des nächsten Zeitpunkts $t+1$, so dass diese Varianten mit geringem Speicher- und Rechenaufwand durchgeführt werden können. Weitere berechnete Werte können in einem anderen Speichermittel abgelegt werden. Die exponentielle Glättung eignet sich daher besonders für den Einsatz in Mikrocontrollern.

Zur Bestimmung eines kurzfristigen Trends entsprechend der Signalableitung kann das Verfahren mit einem relativ großen Glättungsparameter $\alpha^k$ durchgeführt werden,

$$\hat{y}_t^k = 2 \cdot y_t^{k*} - y_t^{k**} , \text{ mit z. B. } \alpha^k = \frac{1}{10}$$

wobei dieser Parameter für die Anwendung in dem jeweiligen Sensor optimiert sein kann.

Eine stark gedämpfte, langfristige Glättung ergibt sich analog zu

$$\hat{y}_t^l = 2 \cdot y_t^{l*} - y_t^{l**}, \text{ mit z. B. } \alpha^l = \frac{1}{1000}$$

wobei der Wert von $\alpha^1$ einer zu optimierenden Zeitkonstante entspricht.

Die für die Angabe eines kurzfristigen Trends notwendige Steigung an einem Punkt $y_t^k$ der geglätteten Kurve berechnet sich nach

$$Y_t^k = \frac{\alpha^k}{1 - \alpha^k} \cdot (y_t^{k*} - y_t^{k**})$$

Dabei lässt sich mit $\alpha = 1/A$ die folgende Vereinfachung bzw. Abschätzung machen:

$$\frac{\alpha}{1-\alpha} = \frac{1/A}{1 - 1/A} = \frac{\frac{1}{A}}{\frac{A-1}{A}} = \frac{1}{A-1} \approx \frac{1}{A} = \alpha \text{ für } \alpha \ll 1 \text{ bzw. } A \gg 1,$$

woraus sich für die Steigung

$$Y_t^k = \alpha^k \cdot (y_t^{k*} - y_t^{k**}) = \frac{1}{10} \cdot (y_t^{k*} - y_t^{k**})$$

ergibt, was sich, abhängig von der Länge des Intervalls zwischen den Messungen durch einen Faktor auf eine beliebige Zeiteinheit skalieren lässt.

Für eine Berechnung der absoluten Änderungen findet eine starke Dämpfung Anwendung, so das eine Trendänderung erst sehr viel später in das geglättete Signal einfließt, abhängig vom Wert von $\alpha^l$, ähnlich einer Zeitkonstante. Aus der Differenz des kurzzeitig und des langzeitig geglätteten Wertes ergibt sich dann der absolute Anstieg

$$\Delta y_t^{abs} = \hat{y}_t^k - \hat{y}_t^l ,$$

wobei hierbei $\hat{y}_t^l$ nicht vollständig wie vorstehend berechnet werden muss, für den gewünschten Effekt mit einer größeren Dämpfung genügt die Berechnung von $\Delta y_t^{abs} = \hat{y}_t^k - y_t^{l**} = W_t$.

$W_t$ ist dabei ein Maß für die absolute Änderung.

Der stark geglättete Wert $\hat{y}_t^l$ folgt dem schwach geglätteten Wert $\hat{y}_t^k$ mit einem zeitlichen Abstand; dieser Abstand wird wiederum durch die Glättungsfaktoren festgelegt. Die Glättungsfaktoren $\alpha^k$ für die schwache Glättung und $\alpha^l$ für die starke Glättung entsprechen kurzen bzw. langen Zeitintervallen bei einer Glättung durch gleitende Mittelwertbildung. Somit kann ohne die Speicherung und Mittelung vieler Zwischenwerte sowohl ein lang- als auch ein kurzfristiger Trend ermittelt werden.

Typische Brände zeichnen sich durch einen schnellen Signalanstieg (große Beträge der kurzfristigen Steigung $Y_t^k$) und eine große Absolutänderung (große Beträge der absoluten Änderung $W_t$ bzw. $\Delta y^{abs}$) aus. Jedoch sind auch sehr langsame Brände denkbar, bei denen trotz einer hohen Absolutänderung nur niedrige Beträge von $Y_t^k$ erreicht werden. Andererseits sollen auch Brände mit einem schnellen Anstieg, bei denen aber nur geringe Absolutwerte erreicht werden, nicht völlig ausgeblendet werden. Deshalb soll für eine Gefahrenerkennung nicht zwingend nur ein einzelnes Signal bewertet werden. Für eine Gefahrenerkennung können neben einzelnen Signalen auch mehrere der geglätteten Signale $\hat{y}_t^k$, $\hat{y}_t^l$, deren Ableitungen und der absoluten Änderung $\Delta y_t^{abs}$ beispielsweise mittels einer Fuzzy-Logik, einer auf Regeln basierten Analyse oder einer Kombination der Signale ausgewertet werden. Für derartige Kombinationen können mehrere Signale, auch abgeleitete Signale, mittels einer Linearkombination bzw. einem Mittelwert beider Steigungen zu einem einzigen Wert $X_t$ als ein Maß für den Trend zusammengefasst werden, der dann mit einem Schwellenwert verglichen werden kann.

Das beschriebene Verfahren löst somit die gestellte Aufgabe, da der Speicherbedarf und die Rechenzeit bei der Bestimmung lang- und kurzfristiger Trends von Sensorsignalen minimiert wurde. Mittels des langfristigen Trends wird es außerdem möglich, trotz der Signaldrift absolute Änderungen der Gaskonzentration für die Alarmierung auszuwerten. Weiterhin kann situationsabhängig der Glättungsfaktor angepasst werden, um beispielsweise nach erfolgter Alarmierung den langfristigen Trend schneller in den Ruhezustand zurückzuführen. Ferner ist es möglich Messsignale auszuwerten, die auch ohne das Einwirken der eigentlich zu messenden Größe bereits stark schwanken bzw. driften ohne den genauen funktionalen Zusammenhang zwischen der Drift und deren Ursache zu kennen. Außerdem kann auch bei bekanntem Zusammenhang zwischen Drift und deren Ursache auf teure zusätzliche Sensoren verzichtet werden.

Die für die Trendberechnungen notwendigen unterschiedlichen Glättungen der Messwerte von Gassensoren, auch mehrerer Gassensoren, können bei einer bevorzugten Variante des Verfahrens im Wesentlichen gleichzeitig durchgeführt werden. Gleichzeitig bedeutet hierbei, dass ein Nutzer des Verfahrens auf die geglätteten Messwerte und Trendberechnungen zeitnah nach der Ermittlung der originalen Signalwerte zugreifen kann.

Eine längerfristige Aufzeichnung von Messwerten mit den Sensoren gestattet bei einer zweckmäßigen Variante des Verfahrens überdies, eine driftbedingte Signaländerung zu identifizieren und von den originalen oder geglätteten Werten abzuziehen und auf diese Weise ein driftkompensiertes Signal zu gewinnen.

Die Aufgabe wird auch gelöst durch eine Vorrichtung zur Erfassung von Konzentrationsänderungen wenigstens eines Zielgases mit einer Messeinrichtung, die wenigstens einen Gassensor aufweist, und mit einer Signalverarbeitungseinrichtung, die dazu eingerichtet ist, das Verfahren nach einem der vorherigen Ansprüche durchzuführen, mit den vorstehend bereits dargelegten Vorteilen.

Besonders geeignet zur Durchführung des Verfahrens ist hierbei eine Ausbildung der Vorrichtung mit wenigstens einem Mikrocontroller als Signalverarbeitungseinrichtung, da bei der Berechnung von geglätteten Werten und Trends verfahrensbedingt jeweils nur zwei alte Datenwerte im Speicher vorgehalten werden müssen und durch die schnelle und aufwandsreduzierte Berechnung in dem Verfahren praktisch jederzeit und zeitnah auf berechnete, aktuelle Werte zugegriffen werden kann.

Zur Erfassung verschiedener Brandformen ist bei einer Weiterbildung der Vorrichtung die Messeinrichtung mit mehreren, auf unterschiedliche Zielgase ansprechenden Gassensoren versehen, für deren Messwerte die vorstehend erwähnten Berechnungen ebenfalls vollständig durchgeführt werden können. Zusätzlich kann die Messeinrichtung mit weiteren Messaufnehmern zur Erfassung von Rauch, der Temperatur, der Luftfeuchtigkeit oder dergleichen physikalischen Parametern versehen sein, deren Werte zu einem oder mehreren weiteren Bewertungskriterien einer Gefahrensituation z. B. mittels eines Brandkenngrößenmustervergleiches verwendet werden können.

Einen besonders zweckmäßigen Einsatz einer oben beschriebenen Vorrichtung stellt die Verwendung in einem Gefahrenmelder, insbesondere einem Brandmelder, dar, welche Verwendung die eingangs gestellte Aufgabe ebenfalls löst.

Das Verfahren wird nachstehend anhand einer einzigen Zeichnungsfigur näher beschrieben, die ein Diagramm mit Mess- und geglätteten Werten sowie Trendberechnungen zeigt. Das Diagramm zeigt den Verlauf verschiedener Signale eines Gassensors vor einem Brand, während eines Brandes und in der Phase nach einem Brand.

Das Diagramm der Figur zeigt dabei ein Koordinatensystem, bei welchem unterschiedliche Ordinaten über eine, eine Zeitachse bildende Abszisse aufgetragen sind. Die Zeitachse weist hierbei einen Sekundenmaßstab auf, während sowohl das in der linken Ordinate aufgetragene Signal eines Sensors als auch der als rechte Ordinate aufgetragene, berechnete Trend in beliebigen Einheiten dargestellt sind.

Auf die linke Ordinate mit der Auftragung der Signalhöhe sind die im Verlauf der Darstellung die mit 1, 2 und 3 bezeichneten Kurven zugeordnet, wobei die mit 1 bezeichnete Kurve die stark schwankenden originalen Messwerte $y_t$ (1) zeigen, die zur Auswertung und Trendbestimmung ungeeignet sind. Gleichzeitig ist die mit 2 bezeichnete Kurve, welche die schwach geglätteten Messwerte $\hat{y}_t^k$ (2) mit $\hat{y}_t^k = 2 \cdot y_t^{k*} - y_t^{k**}$, $y_t^{k*} = \alpha^k \cdot y_t + (1 - \alpha^k) \cdot y_{t-1}^{k*}$ und $y_t^{k**} = \alpha^k \cdot y_t + (1 - \alpha^k) \cdot y_{t-1}^{k**}$ darstellt als helle Kurve über die Kurve 1 gelegt, so dass gut erkennbar ist, dass das aus der Kombination aus einfach und zweifach geglättetem Messsignal gewonnene Signal (2) sehr gut in der Lage ist, das Verhalten der Sensorsignale (1) abzubilden. Die mit 3 bezeichnete Kurve zeigt das stark geglättete Messsignal $\hat{y}_t^l$ mit $\hat{y}_t^l = 2 \cdot y_t^{l*} - y_t^{l**}$, $y_t^{l*} = \alpha^l \cdot y_t + (1 - \alpha^l) \cdot y_{t-1}^{l*}$ und $y_t^{l**} = \alpha^l \cdot y_t + (1 - \alpha^l) \cdot y_{t-1}^{l**}$.

Demgegenüber beziehen sich die "oberen" zwei Kurven 4 und 5 der Darstellung der Zeichnungsfigur auf die rechte Ordinate und geben verschiedene Trends der Messwerte wieder. Die Kurve 5 beschreibt den kurzfristigen Trend $Y_t^k$ (5) der Messwerte. Die bei der entsprechenden Berechnung erfolgte Differenzbildung zwischen einfach und zweifach geglätteten Messwerten entspricht mit $Y_t^k = \alpha^k \cdot (y_t^{k*} - y_t^{k**})$ der Steigung (5) der geglätteten Messwerte (1), während die mit 4 bezeichnete Kurve als Differenz aus mit $\alpha^k$ schwach geglättetem Messwert $\hat{y}_t^k$ (2) und mit $\alpha^l$ stark geglätteten Messwerten $\hat{y}_t^l$ ein Maß für die absolute Änderung (4) $\Delta y_t^{abs} = \hat{y}_t^k - \hat{y}_t^l$ bzw. $W_t = \hat{y}_t^k - y_t^{l**}$ bzw. der geglätteten Messwerte $\hat{y}_t^k$ darstellt.

Die Figur zeigt im Bereich von 0s bis etwa 1000s einen typischen Verlauf der Signale eines driftenden Sensors ohne die Präsenz der zumessenden Größe, einem Brandgas wie z. B. CO, $H_2$ oder $NO_x$. Es ist deutlich zu sehen, dass die Signale 1, 2 und 3 einen klaren Abwärtstrend aufweisen. Dennoch pendelt sich die Kurve der absoluten Änderung (4) auf einen Wert > -100 ein.

Ab einem Abszissenwert von etwa 1000s beginnt ein Feuer. Man erkennt deutlich den sensorbedingten Abfall der Messwerte (1) innerhalb weniger Sekunden um über 1000 Einheiten. Ebenso deutlich erkennt man, dass die mittels zweifacher exponentieller Glättung mit $\alpha^k$ schwach geglättete Kurve 2 aus den werten $\hat{y}_t^k$ (2) der Kurve 1 aus den Messwerten $y_t$ (1) unmittelbar folgt. Die mittels zweifacher exponentieller Glättung mit $\alpha^l$ stark geglättete Kurve 3 aus den Werten $\hat{y}_t^l$ (3) folgt der Kurve 1 jedoch mit einer deutlichen Verzögerung. Aufgrund dieser Verzögerung nimmt die Kurve 4 der absoluten Änderung, die hier als Differenz aus den Werten $\hat{y}_t^l$ (3) und $\hat{y}_t^k$ (2) gebildet ist, schnell einen Wert von -1000 Einheiten an und unterschreitet eine nicht dargestellte Alarmschwelle von z. B. -600 Einheiten. Nach etwa 1500s ist der Brand gelöscht und es setzt eine Erholungsphase ein, in der die Messwerte $y_t$ (1) im Mittel wieder kontinuierlich ansteigen. Dabei erreicht die absolute Änderung (4) jedoch nur Werte von < 200 Einheiten. Dieses Verhalten der Messwerte $y_t$ (1) entspricht dabei durchaus wieder dem normalen Verhalten auch ohne vorhandenes Zielgas. Es wird hier noch darauf hingewiesen, dass der gesamte im Diagramm gezeigten Wertebereich der Messwerte (1) auch im normalen Betrieb des Sensors ohne vorhandenes Zielgas nur durch die Sensordrift erreicht werden kann. Das beschriebene Verfahren bietet also die Möglichkeit mit geringem Aufwand durch Anwendung einer zweifachen exponentiellen Glättung mit geeigneter Wahl der Glättungsparameter $\alpha^k$ und $\alpha^l$ sowie der Alarmschwellen zwischen einer normalen" Signaldrift und echten Gefahrenereignissen zu unterscheiden. Zusätzlich ist es möglich auch bei einem bekannten funktionellen Zusammenhang zwischen einer Sensordrift und deren Ursache auf zusätzliche Sensoren zur Kompensation zu verzichten.

Demgemäß betrifft die Erfindung also ein Verfahren zur Auswertung von Signalen einer Messeinrichtung, die wenigstens einen Gassensor und eine Signalverarbeitungseinrichtung aufweist, welche die Messwerte $y_t$ des wenigstens einen Gassensors verarbeitet. Bei dem Verfahren wird eine mehrfache Glättung, insbesondere eine exponentielle Glättung, auf die durch den mindestens einen Sensor erfassten Messwerte $y_t$ angewandt. Anhand von mit verschiedenen Glättungsparametern durchgeführten Glättungen können kurz- und langfristige Trends der Entwicklung der Messwerte betrachtet und beurteilt werden, außerdem wird ein Maß $W_t$ für die absolute Änderung der geglätteten Messwerte $\hat{y}_t$ gewonnen. Außerdem betrifft die Erfindung eine Vorrichtung, mittels der ein solches Verfahren durchgeführt werden kann sowie die Verwendung einer solchen Vorrichtung in einem Gefahrenmelder.

**Patentansprüche**

1. Verfahren zur Auswertung von Signalen einer Messeinrichtung zur Bestimmung eines Brandes, wobei die Messeinrichtung wenigstens einen Gassensor, und eine Signalverarbeitungseinrichtung aufweist, welche die Messwerte des wenigstens einen Gassensors verarbeitet, **gekennzeichnet durch** zumindest die folgenden Verfahrensschritte:

   a) Vorgabe zumindest eines, bevorzugt mehrerer Glättungsparameter $\alpha^i$;
   b) Erfassung von Messwerten in vorbestimmbaren Zeitintervallen **durch** die Messeinrichtung, Weiterleitung der Messwerte an die Signalverarbeitungseinrichtung und Verarbeitung der Messwerte in dieser;

   c) Bestimmung von Startwerten $y_0$, $y_0^{i*}$ und/oder $y_0^{i**}$ ;
   d) Nach dem Ablauf des nächsten Zeitintervalls Erfassung des Messwerts $y_t$ (1) zum Zeitpunkt $t$;
   e) Anwendung eines Glättungsverfahrens, insbesondere einer exponentiellen Glättung, auf den aktuellen Messwert $y_t$ (1) zur Ermittlung des geglätteten Wertes erster Ordnung $y_t^{i*}$ ;

   f) Erneute Anwendung eines Glättungsverfahrens, insbesondere einer exponentiellen Glättung, auf den Wert $y_t^{i*}$ zur Ermittlung des geglätteten Wertes zweiter Ordnung $y_t^{i**}$ ;

   g) Verrechnung der Werte der ersten und zweiten Glättung $y_t^{i*}$ und $y_t^{i**}$ , insbesondere von kurzfristig geglätteten Werten $y_t^{k*}$ und $y_t^{k**}$ , sowie des Glättungsparameters $\alpha^i$, insbesondere des kurzfristigen Glättungsparameters $\alpha^k$, mittels einer Linearkombination zur Steigung (5), insbesondere der kurzfristigen Steigung $Y_t^k$ im geglätteten Messwert $\hat{y}_t$ (2);
   h) Wiederholung der Verfahrensschritte d)-g) für die Messwerte der Zeitpunkte $t$+1,$t$+2,...,$t$+$n$.

2. Verfahren nach Anspruch 1, **dadurch gekennzeichnet, dass** nach Anwendung des Verfahrens auf Werte $y_t$ (1) nur die geglätteten Werte $y_t^{i*}$ und $y_t^{i**}$ in einem Speicher für die Berechnung der geglätteten Werte des nächsten Zeitpunkts $t$+1 verbleiben.

3. Verfahren nach einem der Ansprüche 1 oder 2, **dadurch gekennzeichnet, dass** die Messwerte in den Verfahrensschritten e) und f) mit insbesondere an den jeweiligen Sensor angepassten Glättungsparametern $\alpha^k$ und/oder $\alpha^l$ geglättet werden und mit diesen die geglätteten Werte $\hat{y}_t^k$ (2) und/oder $\hat{y}_t^l$ (3) bestimmt werden.

4. Verfahren nach Anspruch 3, **dadurch gekennzeichnet, dass** mit der jeweils aktuellen Steigung $Y_t^{k'}$ (5) der Kurve von Punkten $\hat{y}_t^k$ (2) ein kurzfristiger Trend der Messwerte mittels einer Linearkombination von $\alpha^k$, $y_t^{k*}$ und $y_t^{k**}$ bestimmt wird.

5. Verfahren nach Anspruch 4, **dadurch gekennzeichnet, dass** die Werte von $Y_t^k$ (5) mit einem Schwellwert verglichen und abhängig von dem Ergebnis des Vergleichs durch die Messeinrichtung eine Alarmierung ausgelöst wird.

6. Verfahren nach einem oder mehreren der vorhergehenden Ansprüche, **dadurch gekennzeichnet, dass** zur Bestimmung des Brandes, mehrere der geglätteten Signale $\hat{y}_t^k$ , $\hat{y}_t^l$ , deren Ableitungen und eine absolute Änderung $W$, (4) mittels einer Fuzzy-Logik oder einer auf Regeln basierten Analyse oder einer Kombination der Signale ausgewertet werden.

7. Verfahren nach einem der vorhergehenden Ansprüche, **dadurch gekennzeichnet, dass** geglättete Messwerte nach Identifikation eines Driftwertes durch dessen nachträgliche Subtraktion driftkompensiert werden.

8. Vorrichtung zur Erfassung von Messwerten mit einer Messeinrichtung, die wenigstens einen Gassensor aufweist, und mit einer Signalverarbeitungseinrichtung **dadurch gekennzeichnet, dass** die Signalverarbeitungseinrichtung dazu eingerichtet ist, das Verfahren nach einem der vorherigen Ansprüche durchzuführen.

9. Vorrichtung nach Anspruch 8, **dadurch gekennzeichnet, dass** die Signalverarbeitungseinrichtung durch einen Mikrocontroller gebildet ist.

10. Vorrichtung nach einem der Ansprüche 9 oder 8, **dadurch gekennzeichnet, dass** die Messeinrichtung wenigstens einen Gassensor oder mehrere, auf unterschiedliche Zielgase ansprechende Gassensoren aufweist.

11. Vorrichtung nach einem der Ansprüche 8 bis 10, **dadurch gekennzeichnet, dass** die Messeinrichtung mit weiteren Messaufnehmern zur Erfassung der Temperatur, der Luftfeuchtigkeit oder dergleichen physikalischen Parametern versehen ist.

**Claims**

1. Method for evaluating signals of a measuring device for determining a fire, the measuring device having at least one gas sensor and a signal processing device which processes the measured values of the at least one gas sensor, **characterized by** at least the following method steps:

   a) specifying at least one, preferably a plurality of, smoothing parameters $\alpha^i$ ;
   b) determining measured values in predeterminable time intervals by means of the measuring device, passing the measured values onto the signal processing device and processing the measured values in the latter;

   c) determining starting values $y_0$, $y_0^{i*}$ and/or $y_0^{i**}$ ;
   d) detecting the measured value $Y_t$ (1) at the instant $t$ after the expiry of the next time interval;
   e) applying a smoothing method, in particular an exponential smoothing, to the current measured value $y_t$ (1) in order to determine the smoothed value of first order $y_t^{i*}$ ;

   f) reapplying a smoothing method, in particular an exponential smoothing, to the value $y_t^{i*}$ in order to determine the smoothed value of second order $y_t^{i**}$ ;

   g) calculating the values of the first and second smoothing $y_t^{i*}$ and $y_t^{i**}$ , in particular of short-term smoothed values $y_t^{k*}$ and $y_t^{k**}$ , and calculating the smoothing parameter $\alpha^i$, in particular the short-term smoothing parameter $\alpha^k$, by means of a linear combination to form the gradient (5), in particular the short-term gradient $Y_t^k$ , in the smoothed measured value $\hat{y}_t$ (2); and
   h) repeating the method steps d)-g) for the measured values of the instants $t$+1, $t$+2, ..., $t$+$n$.

2. Method according to Claim 1, **characterized in that** after application of the method to values $y_t$ (1) only the smoothed values $y_t^{i*}$ and $y_t^{i**}$ remain in a memory for the calculation of the smoothed values of the next instant t+1.

3. Method according to either of Claims 1 and 2, **characterized in that** the measured values in method steps e) and f) are smoothed with the aid of smoothed parameters $\alpha^k$ and/or $\alpha^l$, adapted in particular to the respective sensor, and the smoothed values $\hat{y}_t^k$ (2) and/or $\hat{y}_t^l$ (3) are determined therewith.

4. Method according to Claim 3, **characterized in that** a short-term trend of the measured values is determined by means of a linear combination of $\alpha^k$, $y_t^{k*}$ and $y_t^{k**}$ and with the aid of the respective current gradient $Y_t^k$ (5) of the curve of points $\hat{y}_t^k$ (2).

5. Method according to Claim 4, **characterized in that** the values of $Y_t^k$ (5) are compared with a threshold value, and an alarm is triggered by the measuring device as a function of the result of the comparison.

6. Method according to one or more of the preceding claims, **characterized in that** in order to determine the fire, a plurality of the smoothed signals $\hat{y}_t^k$, $\hat{y}_t^l$, the derivatives thereof and an absolute change $W_t$ (4) are evaluated by means of a fuzzy logic or a rule-based analysis, or a combination of the signals.

7. Method according to one of the preceding claims, **characterized in that** smoothed measured values are drift-compensated by subsequently subtracting a drift value after identification thereof.

8. Device for detecting measured values with the aid of a measuring device which has at least one sensor, and with the aid of a signal processing device, **characterized in that** the signal processing device is set up to carry out the method according to one of the preceding claims.

9. Device according to Claim 8, **characterized in that** the signal processing device is formed by a microcontroller.

10. Device according to either of Claims 9 and 8, **characterized in that** the measuring device has at least one gas sensor or a plurality of gas sensors responding to different target gases.

11. Device according to one of Claims 8 to 10, **characterized in that** the measuring device is provided with further measuring sensors for detecting temperature, air humidity or physical parameters of such type.

**Revendications**

1. Procédé d'évaluation de signaux d'un dispositif de mesure permettant de détecter un incendie, lequel dispositif de mesure présente au moins un capteur de gaz et un dispositif de traitement de signaux qui traite les valeurs de mesure dudit au moins un capteur de gaz, **caractérisé par** au moins les étapes de procédé suivantes :

a) prescription d'au moins un, de préférence plusieurs paramètres de lissage $\alpha^i$;
b) saisie de valeurs de mesure à des intervalles de temps prédéterminables par le dispositif de mesure, transmission des valeurs de mesure au dispositif de traitement de signaux et traitement des valeurs de mesure dans celui-ci ;

c) détermination de valeurs de départ $y_0$, $y_0^{i*}$ et/ou $y_0^{i**}$ ;
d) après expiration de l'intervalle de temps suivant, saisie de la valeur de mesure $y_t$ (1) à l'instant $t$ ;
e) application d'un procédé de lissage, en particulier d'un lissage exponentiel, sur la valeur de mesure actuelle $y_t$ (1) pour déterminer la valeur lissée de premier ordre $y_t^{i*}$ ;

f) application renouvelée d'un procédé de lissage, en particulier d'un lissage exponentiel, sur la valeur $y_t^{i*}$ pour déterminer la valeur lissée de deuxième ordre $y_t^{i**}$ ;

g) combinaison des valeurs du premier et du deuxième lissage $y_t^{i*}$ et $y_t^{i**}$, en particulier de valeurs lissées à court terme $y_t^{k*}$ et $y_t^{k**}$, ainsi que du paramètre de lissage $\alpha^i$, en particulier du paramètre de lissage à court terme $\alpha^k$, au moyen d'une combinaison linéaire pour calculer la pente (5), en particulier la pente à court terme $Y_t^k$ à la valeur de mesure lissée $\hat{y}_t$ (2) ;

h) répétition des étapes de procédé d) à g) pour les valeurs de mesure des instants $t + 1$, $t + 2$, ..., $t + n$.

2. Procédé selon la revendication 1, **caractérisé en ce qu'**après application du procédé aux valeurs $y_t$ (1), seules les valeurs lissées $y_t^{i*}$ et $y_t^{i**}$ restent dans une mémoire pour le calcul des valeurs lissées de l'instant suivant $t + 1$.

3. Procédé selon une des revendications 1 ou 2, **caractérisé en ce que** les valeurs de mesure aux étapes de procédé e) et f) sont lissées avec des paramètres de lissage $\alpha^k$ et/ou $\alpha^l$ adaptés en particulier au capteur respectif et avec lesquels les valeurs lissées $\hat{y}_t^k$ (2) et/ou $\hat{y}_t^l$ (3) sont déterminées.

4. Procédé selon la revendication 3, **caractérisé en ce qu'**une tendance à court terme des valeurs de mesure est déterminée avec la pente actuelle $Y_t^k$ (5) de la courbe de points $\hat{y}_t^k$ (2) au moyen d'une combinaison linéaire de $\alpha^k$, $y_t^{k*}$ et $y_t^{k**}$.

5. Procédé selon la revendication 4, **caractérisé en ce que** les valeurs de $Y_t^k$ (5) sont comparées à une valeur seuil et une alarme est déclenchée par le dispositif de mesure en fonction du résultat de la comparaison.

6. Procédé selon une ou plusieurs des revendications précédentes, **caractérisé en ce que** pour détecter un incendie, plusieurs des signaux lissés $\hat{y}_t^k$, $\hat{y}_t^l$, leurs dérivées et une variation absolue $W_t$ (4) sont évalués au moyen d'une logique floue, d'une analyse basée sur des règles ou d'une combinaison des signaux.

7. Procédé selon une des revendications précédentes, **caractérisé en ce qu'**après identification d'une valeur de dérive, les valeurs lissées sont compensées en dérive par soustraction ultérieure de celle-ci.

8. Dispositif pour saisir des valeurs de mesure avec un dispositif de mesure qui présente au moins un capteur de gaz et avec un dispositif de traitement de signaux, **caractérisé en ce que** le dispositif de traitement de signaux est conçu pour réaliser le procédé selon une des revendications précédentes.

9. Dispositif selon la revendication 8, **caractérisé en ce que** le dispositif de traitement de signaux est formé par un microcontrôleur.

10. Dispositif selon une des revendications 9 ou 8, **caractérisé en ce que** le dispositif de mesure présente au moins un capteur de gaz ou plusieurs capteurs de gaz réagissant à différents gaz cibles.

11. Dispositif selon une des revendications 8 à 10, **caractérisé en ce que** le dispositif de mesure est pourvu d'autres capteurs pour détecter la température, l'humidité de l'air ou des paramètres physiques analogues.

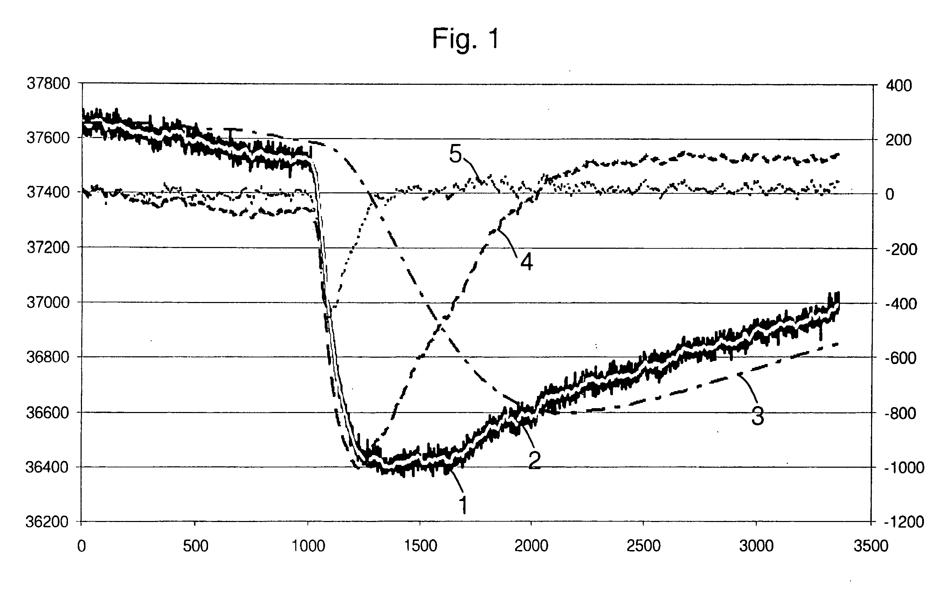

## Fig. 1

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Nicht-Patentliteratur**

- Experimental Analysis of the Performance of a Multisensor Two-stage Fire Detection and Water Discharge Algorithm. *Fire Safety Journal,* vol. 29

- **VON E. GARDNER.** *Forecasting with exponential smoothing: Some Guidelines for Model Selection*